# EUROPEAN PATENT APPLICATION

(11) **EP 4 810 020 A1**
(43) Date of publication of application: **23.09.2026**
(21) Application number: 24890040.9
(22) Date of filing: 29.05.2024
(51) Int. Cl.: A61K 9/127, A61K 47/24, A61K 47/14, A61P 35/00

(54) **NANOPARTICLE ADIPOSOME ENCAPSULATING HYDROPHOBIC SMALL-MOLECULE DRUG, PREPARATION METHOD THEREFOR, AND USE THEREOF**

(30) Priority: 14.11.2023 CN 202311512646
(71) Applicant: WeCareLife Biotech Co., Ltd., Beijing 102629 (CN)
(72) Inventor: CAO, Zhen, Beijing 100101 (CN); ZHOU, Chang, Beijing 100101 (CN); ZHI, Zelun, Beijing 100101 (CN); PAN, Bin, Beijing 100101 (CN)
(74) Representative: Eisenführ Speiser
(86) International application number: PCT/CN2024/095933
(87) International publication number: WO 2025/102670

(57) **Abstract**

A nanoparticle adiposome encapsulating a hydrophobic small-molecule drug, a preparation method therefor, and a use thereof. The nanoparticle adiposome encapsulating the hydrophobic small-molecule drug comprises a monomolecular phospholipid membrane and hydrophobic small-molecule drug-containing neutral lipids encapsulated within the monomolecular phospholipid membrane. The nanoparticle adiposome has the hydrophobic small-molecule drug-containing neutral lipids as a hydrophobic core, and nanobeads wrapped by the monomolecular phospholipid membrane can efficiently dissolve and encapsulate a hydrophobic small-molecule compound. In addition, the adiposome encapsulating the hydrophobic small-molecule drug has bioactivity, can remarkably kill cancer cells, and has a killing effect superior to that of a free drug. In addition, the preparation method is simple and efficient. Therefore, the adiposome is a prospecting hydrophobic small-molecule drug delivery platform, and can be widely applied to the treatment of diseases such as cancer, infectious diseases and metabolic diseases.

## Description

This application claims the priority of Chinese Patent Application No. 202311512646.3, filed with the China National Intellectual Property Administration on November 14, 2023, and titled with "NANOPARTICLE ADIPOSOME ENCAPSULATING HYDROPHOBIC SMALL-MOLECULE DRUG, PREPARATION METHOD THEREFOR, AND USE THEREOF", which is hereby incorporated by reference in its entirety.

### FIELD

The present disclosure relates to the field of pharmaceutical technology, and in particular to an adiposome nanoparticle encapsulating a hydrophobic small molecule drug, and a production method and use thereof.

### BACKGROUND

Small molecule drugs are an important means for treating various diseases. Hydrophobic small molecule drugs refer to small molecule compounds that are insoluble or poorly soluble in water. Due to their hydrophobicity (i.e., poor solubility in water or other polar solvents), these drugs often have low solubility and bioavailability in biological systems. Many common drugs belong to hydrophobic small molecule drugs, such as antibiotics, anticancer drugs, lipid-lowering drugs, and the like. In addition, 90% of small molecule drugs under development belong to hydrophobic small molecule compounds. Due to the poor water solubility, their absorption and bioavailability are seriously affected, and varying degrees of toxic or side effects are caused, thereby seriously limiting their clinical applications. Therefore, improving the solubility of hydrophobic small molecule drugs is an important challenge in drug development.

To improve the solubility and bioavailability of hydrophobic small molecule drugs, the following methods are often employed: 1) drug modification, which involves chemically modifying the structure of hydrophobic small molecule drugs, for example, by adding polar functional groups; 2) use of cosolvents, which involves adding some organic solvents and surfactants to increase the solubility of hydrophobic drugs, such as ethanol, Tween 80, polyoxyethylated castor oil, and the like; and 3) drug carriers, which utilize lipid nanocarriers to encapsulate and deliver hydrophobic small molecule drugs, such as liposomes, polymer nanoparticles, and nanomicelles. However, drug modification often results in loss of activity of small molecule drugs. Surfactants such as ethanol and Tween 80 as excipients often cause toxic side effects such as hemolysis and allergies.

In addition, although there are various delivery carriers for encapsulating and delivering hydrophobic small molecule compounds, there are also some drawbacks, such as: 1) liposomes, which are prepared by simple methods such as mixing, shaking, and sonication; however, liposomes have poor stability and are prone to fusion, rupture, or drug leakage, which affects their distribution in vivo and drug release; 2) lipid microspheres, which are prepared by emulsion-solvent evaporation, coprecipitation, solution polymerization, and the like; however, these methods may require the use of organic solvents, which may present the problem of solvent residue; in addition, the preparation process of lipid microspheres affects the stability of the drug; and 3) solid lipid nanoparticles: solid lipid nanoparticles are prepared through melting methods, solvent methods, supercritical fluid methods, and the like, and the preparation process requires high temperature or organic solvents, which may cause drug crystallization or separation, thereby affecting their stability.

### SUMMARY

In view of the foregoing, the technical problem to be solved by the present disclosure is to provide an adiposome nanoparticle encapsulating a hydrophobic small molecule drug, and a preparation method and use thereof. The prepared drug-loaded adiposome nanoparticle can efficiently dissolve and encapsulate hydrophobic small molecule compounds.

To achieve the above objects, one of the objects of the present disclosure is to provide an adiposome nanoparticle encapsulating a hydrophobic small molecule drug, comprising monolayer phospholipid membrane and a neutral lipid containing the hydrophobic small molecule drug, wherein the neutral lipid is encapsulated within the monolayer phospholipid membrane.

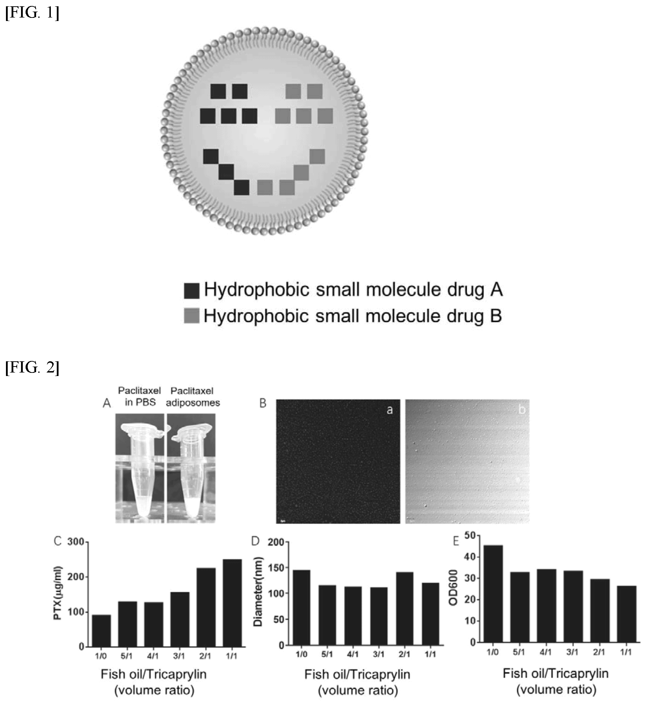

The structural schematic diagram of the adiposome nanoparticle encapsulating a hydrophobic small molecule drug provided by the present disclosure is shown in FIG. 1.

As shown in FIG. 1, the adiposome nanoparticle encapsulating a hydrophobic small molecule drug provided by the present disclosure has a hydrophobic core formed by a neutral lipid (shown as the light gray portion in FIG. 1), which can effectively encapsulate one or more hydrophobic small molecule drugs (shown as hydrophobic small molecule drug A and hydrophobic small molecule drug B in FIG. 1), improving the solubility and bioavailability of the hydrophobic small molecule drugs. The neutral lipid is wrapped by a monolayer phospholipid membrane to form a nanoparticle. The adiposome nanoparticle is similar in structure to naturally occurring lipid droplets and lipoproteins, and can efficiently dissolve and encapsulate hydrophobic small molecule compounds. In addition, the components of the adiposome naturally exist in the body and have good biocompatibility. Therefore, the adiposome is a promising delivery platform for hydrophobic small molecule drugs and can be widely used in the treatment of diseases such as cancers, infectious diseases, and metabolic diseases.

The adiposome nanoparticle encapsulating a hydrophobic small molecule drug provided by the present disclosure is suitable for most hydrophobic small molecule drugs, including but not limited to one or more of paclitaxel, honokiol, camptothecin, auristatin derivative MMAE, and the like.

The adiposome nanoparticle encapsulating a hydrophobic small molecule drug provided by the present disclosure can encapsulate one hydrophobic small molecule drug or simultaneously encapsulate a plurality of hydrophobic small molecule drugs, such as paclitaxel-camptothecin, and the like.

Optionally, the neutral lipid is selected from one or more of fish oil, corn oil, tricaprylin, and retinyl ester.

In some specific embodiments of the present disclosure, the neutral lipid is fish oil or corn oil.

In some specific embodiments of the present disclosure, the neutral lipid is a mixture of fish oil and tricaprylin. Optionally, the fish oil and the tricaprylin have a volume ratio of (1 to 5):(1 to 2); more preferably (1 to 5):1, specifically 5:1, 4:1, 3:1, 2:1, 1:1, and the like.

In some specific embodiments of the present disclosure, the neutral lipid is a mixture of fish oil and retinyl ester. Optionally, the fish oil and the retinyl ester have a volume ratio of (1 to 5):(1 to 2); specifically 5:1, 4:1, 3:1, 2:1, 1:1, 1:2, and the like.

In some specific embodiments of the present disclosure, the neutral lipids area mixture of tricaprylin and corn oil. Optionally, the tricaprylin and the corn oil have a volume ratio of (1 to 5):(1 to 2); preferably (1 to 5):1, specifically 5:1, 4:1, 3:1, 2:1, 1:1, and the like.

In some specific embodiments of the present disclosure, the neutral lipid is a mixture of fish oil, tricaprylin, and retinyl ester. Optionally, the fish oil, the tricaprylin, and the retinyl ester have a volume ratio of (2 to 4):(1 to 3):(1 to 2), specifically 4:1:1, 3:2:1, 3:1:2, 2:3:1, 2:2:2, and the like.

Optionally, the phospholipid membrane is selected from one or more of 2-di-(9Z-octadecenoyl)-sn-glycero-3-phosphocholine, phosphatidyl choline (PC), phosphatidic acid (PA), phosphatidyl inositol (PI), phosphatidyl ethanolamine (PE), and PEGylation phosphatidyl ethanolamine (PEG-PE).

Optionally, the phospholipid membrane is selected from a mixture of phosphatidyl choline and any one of 2-di-(9Z-octadecenoyl)-sn-glycero-3-phosphocholine, phosphatidic acid, phosphatidyl inositol, phosphatidyl ethanolamine, or PEGylation phosphatidyl ethanolamine.

Wherein the molar content of 2-di-(9Z-octadecenoyl)-sn-glycero-3-phosphocholine, phosphatidic acid, phosphatidyl inositol, phosphatidyl ethanolamine, or PEGylation phosphatidyl ethanolamine in the mixture is 5% to 10%.

Optionally, the phospholipid membrane is selected from phosphatidic acid and phosphatidyl choline. The molar ratio of the phosphatidic acid to the phosphatidyl choline is (5 to 10):(90 to 95).

Optionally, the phospholipid membrane is selected from phosphatidyl inositol and phosphatidyl choline. The molar ratio of the phosphatidyl inositol to the phosphatidyl choline is (5 to 10):(90 to 95).

Optionally, the phospholipid membrane is selected from phosphatidyl ethanolamine and phosphatidyl choline. The molar ratio of the phosphatidyl ethanolamine to the phosphatidyl choline is (5 to 10):(90 to 95).

Optionally, the phospholipid membrane is selected from PEGylation phosphatidyl ethanolamine and phosphatidyl choline. The molar ratio of the PEGylation phosphatidyl ethanolamine to the phosphatidyl choline is (5 to 10):(90 to 95).

The adiposome nanoparticle encapsulating a hydrophobic small molecule drug provided by the present disclosure has a uniform spherical structure.

Test results show that when encapsulating the hydrophobic small molecule drug paclitaxel, using fish oil and tricaprylin as the neutral lipids, especially when the volume ratio of the two is 1:1, a higher drug loading concentration can be achieved, reaching 230 µg/mL. The particle size of the prepared paclitaxel-loaded adiposome nanoparticles is about 130 nm. Using fish oil and retinyl ester as the neutral lipids, the loading concentration is about 400 µg/mL and the particle size is about 200 nm; especially when the volume ratio of the two is 1:2, a higher drug loading concentration can be achieved, reaching 600 µg/mL, and the particle size of the prepared paclitaxel-loaded adiposome nanoparticles is about 800 nm. Using a mixture of fish oil, tricaprylin, and retinyl ester as the neutral lipids, the loading concentration of paclitaxel is about 600 µg/mL and the particle size is about 200 nm.

Test results show that the selection of phospholipid also affects the drug loading concentration and particle size. Taking paclitaxel as an example, when PE or PEG-PE is added as a phospholipid, the content of paclitaxel in the paclitaxel adiposome is increased, and the loading rate can reach more than 500 µg/mL. The addition of PI, PE, and PEG-PE increases the average particle size of the paclitaxel adiposome, with a particle size of about 200 to 300 nm. When 10% of PE is added, the concentration of the paclitaxel adiposome is greatly increased, and OD600 can reach 120.

When encapsulating honokiol in the present disclosure, triolein is used as the neutral lipid, and 2-di-(9Z-octadecenoyl)-sn-glycero-3-phosphocholine is used as the phospholipid. The loading concentration of honokiol is 17 µg/mL, and the particle size is about 114.2 nm.

When encapsulating camptothecin in the present disclosure, tricaprylin or a mixture of fish oil and tricaprylin is used as the neutral lipid. Optionally, the volume ratio of the fish oil to the tricaprylin is 1:1, and 2-di-(9Z-octadecenoyl)-sn-glycero-3-phosphocholine is used as the phospholipid. The loading concentration of camptothecin is 4 mg/L, and the particle size is about 120 nm.

When encapsulating the auristatin derivative MMAE in the present disclosure, triolein or corn oil is used as the neutral lipid, and 2-di-(9Z-octadecenoyl)-sn-glycero-3-phosphocholine is used as the phospholipid. When corn oil is used as the neutral lipids, the loading concentration of MMAE is 100 µg/mL, and when tricaprylin is used as the neutral lipid, the loading concentration of MMAE is 40 µg/mL; the particle size of the prepared MMAE-loaded adiposome nanoparticles is about 140 nm.

When co-encapsulating paclitaxel and camptothecin in the present disclosure, a mixture of fish oil and tricaprylin is used as the neutral lipid. Optionally, the volume ratio is 1:1, and 2-di-(9Z-octadecenoyl)-sn-glycero-3-phosphocholine is use as the phospholipid. Test results show that the loading concentration of paclitaxel is 65 mg/L, the loading concentration of camptothecin is 4 mg/L, and the average particle size of the prepared adiposome nanoparticles is about 115.5 nm.

Test results show that after the hydrophobic small molecule drug is prepared into adiposome nanoparticles using the above neutral lipids, the hydrophobic small molecule drug, which originally had very low solubility in the solvent, can present a uniform milky white solution state, indicating that its solubility is greatly improved.

The second object of the present disclosure is to provide a method for producing the above-mentioned adiposome nanoparticle encapsulating a hydrophobic small molecule drug, comprising steps of:
S1) mixing a solution of a hydrophobic small molecule drug with a neutral lipid, and removing solvent to obtain a neutral lipid containing the hydrophobic small molecule drug;
S2) mixing the neutral lipid containing the hydrophobic small molecule drug with a phospholipid to obtain a lipid mixture, and performing separation to obtain the adiposome nanoparticle encapsulating the hydrophobic small molecule drug.

Optionally, the solvent of the hydrophobic small molecule drug solution is selected from a solvent capable of dissolving a hydrophobic small molecule drug such as absolute ethanol, chloroform, methanol, acetone, and the like.

Optionally, the concentration of the hydrophobic small molecule drug solution is 0 to 40 mg/mL. In some specific embodiments, the concentration of the hydrophobic small molecule drug solution is 20 mg/mL.

The concentration of the hydrophobic small molecule drug solution may also be adjusted according to the actual situation of the drug.

Optionally, the ratio of the hydrophobic small molecule drug solution to the neutral lipid may be adjusted according to the actual situation. Taking paclitaxel as an example, the ratio of the paclitaxel to the neutral lipid may be 1:1 to 1:5.

Optionally, the method for removing the solvent in the step S1) is drying by blowing with high-purity nitrogen gas.

First, phospholipid solution is placed in a centrifuge tube, and the solvent is dried by blowing with high-purity nitrogen gas; then the neutral lipid containing the hydrophobic small molecule drug is added to achieve mixing of the two.

Optionally, the step S2) is specifically:
A) vortexing the neutral lipid containing the hydrophobic small molecule drug and the phospholipid in a buffer to obtain a milky white lipid mixture;
B) repeatedly centrifuging and vortexing the lipid mixture, and performing separation to obtain the adiposome nanoparticle encapsulating the hydrophobic small molecule drug.

The buffer includes but is not limited to phosphate buffer, HEPES buffer, sucrose solution, NaCl solution, KCl solution, MgCl₂ solution, and the like.

Optionally, the vortexing in the step A) is performed for 3 to 7 min, and at a rotation speed of 3000 to 4000 rpm.

Optionally, the obtained milky white lipid mixture is purified. In the present disclosure, the lipid mixture is purified by repeatedly centrifuging and vortexing. The centrifuging and vortexing may be repeated 1 to 3 times.

Optionally, the obtained milky white lipid mixture is subjected to a first centrifugation. Optionally, the first centrifugation is performed at a speed of 800 to 1200 g for 3 to 7 min. After centrifugation, the liquid phase system presents two layers, and the lower milky white solution is collected by extraction and vortexed to obtain a milky white lipid mixture 2.

Optionally, the lipid mixture 2 is subjected to a second centrifugation. Optionally, the second centrifugation is performed at a speed of 18000 to 22000 g for 3 to 7 min. After centrifugation, the precipitate component at the bottom of the centrifuge tube is removed, and vortexing is performed to obtain a milky white lipid mixture 3.

Optionally, the lipid mixture 3 is subjected to a third centrifugation. Optionally, the third centrifugation is performed at a speed of 800 to 1200 g for 3 to 7 min. After centrifugation, the liquid phase system presents two layers, and the lower milky white solution is collected by extraction and vortexed to obtain a pure milky white lipid mixture.

The third object of the present disclosure is to provide a use of the above-mentioned adiposome nanoparticle encapsulating a hydrophobic small molecule drug in manufacture of a medicament for preventing, alleviating, or treating a tumor, an infectious disease, and/or a metabolic disease.

The fourth object of the present disclosure is to provide a use of the above adiposome nanoparticle encapsulating a hydrophobic small molecule drug for preventing, alleviating, or treating a tumor, an infectious disease, and/or a metabolic disease.

Cell test results show that the adiposome nanoparticle encapsulating a hydrophobic small molecule drug produced by the present disclosure has biological activity and can significantly kill a cancer cell, and the killing effect is superior to that of a free drug.

Optionally, the above cancer cell is one or more of human embryonic kidney epithelial cells HEK293, human breast cancer cells MCF7, and human breast cancer cells MDA-MB-231.

Optionally, the tumor is one or more of kidney cancer, breast cancer, and lung cancer.

Optionally, the infectious disease is one or more of influenza and tuberculosis.

Optionally, the metabolic disease is one or more of diabetes, obesity, and cardiovascular diseases.

The fifth object of the present disclosure is to provide a medicament for alleviating or treating a tumor, an infectious disease, and/or a metabolic disease, comprising the above-mentioned adiposome nanoparticle encapsulating a hydrophobic small molecule drug.

Compared with the prior art, the present disclosure provides an adiposome nanoparticle encapsulating a hydrophobic small molecule drug, comprising a monolayer phospholipid membrane and a neutral lipid containing the hydrophobic small molecule drug encapsulated within the monolayer phospholipid membrane. The novel adiposome nanoparticle provided by the present disclosure has a neutral lipid containing a hydrophobic small molecule drug as a hydrophobic core, which is a nanoparticle encapsulated by a monolayer phospholipid membrane, and can efficiently dissolve and encapsulate a hydrophobic small molecule compound. In addition, the adiposome encapsulating a hydrophobic small molecule drug has biological activity and can significantly kill cancer cells, and the killing effect is superior to that of a free drug. Furthermore, the production method is simple and efficient. Therefore, the adiposome is a promising delivery platform for hydrophobic small molecule drugs and can be widely used in the treatment of a disease such as a cancer, an infectious disease, and a metabolic disease.

### BRIEF DESCRIPTION OF DRAWINGS

FIG. 1 is a structural schematic diagram of an adiposome nanoparticle encapsulating a hydrophobic small molecule drug provided by the present disclosure;
FIG. 2 shows the test results of adiposomes encapsulating hydrophobic small molecule compound paclitaxel prepared in 1.1 of Example 1;
FIG. 3 shows the test results of adiposomes encapsulating hydrophobic small molecule compound paclitaxel prepared in 1.2 of Example 1;
FIG. 4 shows the test results of adiposomes encapsulating hydrophobic small molecule compound paclitaxel prepared in 1.3 of Example 1;
FIG. 5 shows the test results of adiposomes encapsulating hydrophobic small molecule compound paclitaxel prepared in 1.4 of Example 1;
FIG. 6 shows the test results of adiposomes encapsulating hydrophobic small molecule compound paclitaxel prepared in 1.5 of Example 1;
FIG. 7 shows the test results of adiposomes encapsulating hydrophobic small molecule compound honokiol prepared in Example 2;
FIG. 8 shows the test results of adiposomes encapsulating hydrophobic small molecule compound camptothecin prepared in Example 3;
FIG. 9 shows the test results of adiposomes encapsulating hydrophobic small molecule compound auristatin derivative MMAE prepared in Example 4;
FIG. 10 shows the test results of adiposomes simultaneously encapsulating hydrophobic small molecule compounds paclitaxel and camptothecin prepared in Example 5;
FIG. 11 shows the comparison of antitumor performance of paclitaxel adiposomes and free paclitaxel;
FIG. 12 shows the comparison of cell viability treated with honokiol adiposomes or free honokiol;
FIG. 13 shows the comparison of cell viability treated with MMAE adiposomes or free MMAE.

### DETAILED DESCRIPTION

To further illustrate the present disclosure, the adiposome nanoparticle encapsulating a hydrophobic small molecule drug and the production method and use thereof provided by the present disclosure is described in detail in conjunction with the examples.

The hydrophobicity of a drug is generally measured by the oil-water partition coefficient (logP). The logP value refers to the logarithm of the ratio of the partition coefficient of a substance in n-octane (oil) and water, reflecting the distribution of the substance in the oil-water phases. The larger the logP value, the more lipophilic and hydrophobic the substance is. To demonstrate that the adiposome is a platform capable of encapsulating and delivering hydrophobic small molecule drugs, in the present disclosure, some hydrophobic small molecule compounds with logP values greater than 1.5 (shown in Table 1), such as paclitaxel, honokiol, camptothecin, and auristatin derivative MMAE (Monomethyl auristatin E), were selected for encapsulation and corresponding biological activity verification.

**Table 1 logP values of hydrophobic small molecule drugs encapsulated in adiposomes**

| **Hydrophobic small molecule drug** | **logP** |
|---|---|
| Paclitaxel | 3.2 |
| Honokiol | 5.0 |
| Auristatin derivative MMAE (Monomethyl auristatin E) | 3.4 |
| Camptothecin | 1.9 |

### Example 1 Construction of adiposomes encapsulating hydrophobic small molecule compound paclitaxel (paclitaxel adiposomes)

1.1. Adiposomes encapsulating hydrophobic small molecule compound paclitaxel were prepared by altering the neutral lipid conditions, specifically the ratio of fish oil and tricaprylin (8:0TAG).
1.1.1) Neutral lipids were prepared according to the following 6 volume ratios, i.e., fish oil/tricaprylin = 1/0, 5/1, 4/1, 3/1, 2/1, and 1/1;
1.1.2) Paclitaxel (PTX) was dissolved in absolute ethanol at a concentration of 20 mg/mL;
1.1.3) 200 µL of the above neutral lipids prepared with different volume ratios were taken and 200 µL of paclitaxel solution (PTX) was added, and ethanol was dried by blowing with high-purity nitrogen gas to obtain neutral lipids containing PTX;
1.1.4) 80 µL of 2-di-(9Z-octadecenoyl)-sn-glycero-3-phosphocholine solution (containing 2 mg DOPC) was added to a microcentrifuge tube, and the solvent was dried by blowing with high-purity nitrogen gas;
1.1.5) 100 µL of PBS and 5 µL of the neutral lipids containing PTX prepared in step 1.1.3) were added to the microcentrifuge tube, respectively, and vortexed for 4 min (vortex for 10 s, and pause for 10 s) (in actual applications, vortexing for 3 to 7 min is acceptable, the vortexing may be performed at 3000 to 4000 rpm, and 4000 rpm was used in this example) to obtain a milky white lipid mixture 1. The lipid mixture 1 was centrifuged at 1000 g for 5 min (in actual applications, centrifugation at 800 to 1200 g for 3 to 7 min is acceptable). After centrifugation, the liquid phase system presented two layers, and the lower milky white solution was collected by extraction and vortexed to obtain a milky white lipid mixture 2.
1.1.6) The lipid mixture 2 obtained in step 1.1.5) was centrifuged at 20000 g for 5 min (in actual applications, centrifugation at 18000 to 22000 g for 3 to 7 min is acceptable). After centrifugation, the precipitate component at the bottom of the microcentrifuge tube was removed, and vortexing was performed to obtain a milky white lipid mixture 3.
1.1.7) The lipid mixture 3 obtained in step 1.1.6) was centrifuged at 1000 g for 5 min (in actual applications, centrifugation at 800 to 1200 g for 3 to 7 min is acceptable). After centrifugation, the liquid phase system presented two layers, and the lower milky white solution was collected by extraction and vortexed to obtain a milky white lipid mixture 4, which was the final adiposome encapsulating hydrophobic small molecule compound paclitaxel (paclitaxel adiposome).

Paclitaxel and the prepared paclitaxel adiposomes were dissolved in PBS, respectively. The dissolution picture is shown in FIG. 2A, which shows the morphology of paclitaxel in PBS and adiposomes in PBS. Paclitaxel, due to its hydrophobicity, has very low solubility in PBS and presented a turbid state, while paclitaxel encapsulated in adiposomes presented a uniform milky white solution state.

The prepared paclitaxel adiposomes were further observed by laser confocal microscopy. The paclitaxel adiposomes presented a uniform spherical structure (see FIG. 2B, where a is the result of paclitaxel adiposomes stained red by the neutral lipid-specific dye LipidTox Red, and b is the DIC (Differential Interference Contrast) result of paclitaxel adiposomes, with a scale bar of 2 µm).

The paclitaxel adiposomes were dissolved in methanol (chromatographic grade) for sample preparation, and the content of PTX in the paclitaxel adiposomes was measured by HPLC. The detection conditions were: chromatographic column Agilent Zorbax SB-C18, mobile phase methanol:acetonitrile:water = 4:3:3 (volume ratio), column temperature 25°C, detection wavelength 227 nm, flow rate 1 mL/min. When the volume ratio of fish oil to tricaprylin was 1:1, the content of PTX in the prepared paclitaxel adiposomes was the highest, reaching 230 µg/mL, while under other ratio conditions, the content of PTX in the paclitaxel adiposomes was relatively low (see FIG. 2C, which is a diagram of the content of PTX in paclitaxel adiposomes measured by high performance liquid chromatography). The average particle size of paclitaxel adiposomes prepared with different neutral lipid volume ratios was not significantly different, about 130 nm (see FIG. 2D, which is a diagram of the average particle size of paclitaxel adiposomes measured by dynamic light scattering particle size analyzer). When the volume ratio of fish oil to tricaprylin was 1:0, the concentration of the prepared paclitaxel adiposomes was the highest, with an OD600 of about 45, while under other ratio conditions, the concentrations of paclitaxel adiposomes were not significantly different, with OD600 of about 30 (see FIG. 2E, which is a diagram of the concentration of paclitaxel adiposomes measured by a multifunctional microplate reader, expressed as OD600).

1.2. Adiposomes encapsulating hydrophobic small molecule compound paclitaxel were prepared by altering the neutral lipid conditions, specifically the ratio of fish oil and retinyl ester.

1.2.1) Neutral lipids were prepared according to the following 7 volume ratios, i.e., fish oil/retinyl ester = 1/0, 5/1, 4/1, 3/1, 2/1, 1/1, and 1/2.

The subsequent steps were the same as the preparation method in 1.1 above.

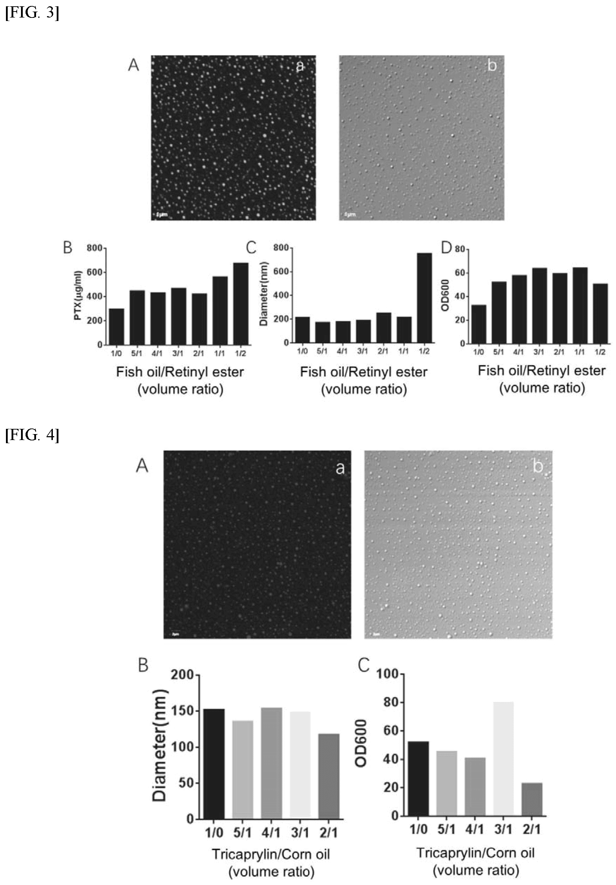

The obtained paclitaxel adiposomes were observed by laser confocal microscopy. The experimental results showed that when neutral lipid conditions were changed, adiposomes encapsulating paclitaxel could also be prepared using fish oil and retinyl ester. The paclitaxel adiposomes presented a uniform spherical structure (see FIG. 3A, where a is the result of paclitaxel adiposomes stained green by the neutral lipid-specific dye LipidTox Green, and b is the DIC result of paclitaxel adiposomes, with a scale bar of 1 µm). The content of PTX in paclitaxel adiposomes was measured by HPLC. When the volume ratio of fish oil to retinyl ester was 1:2, the content of PTX in the prepared paclitaxel adiposomes was the highest, reaching 600 µg/mL, and the average particle size was the largest, about 800 nm. Under other ratio conditions, the content of PTX in paclitaxel adiposomes was relatively low, about 400 µg/mL (see FIG. 3B, which is a diagram of the content of PTX in paclitaxel adiposomes measured by high performance liquid chromatography), and the average particle size was not significantly different, about 200 nm (see FIG. 3B and 3C, where C is a diagram of the average particle size of paclitaxel adiposomes measured by dynamic light scattering particle size analyzer). The concentrations of paclitaxel adiposomes prepared under different neutral lipid ratio conditions were not significantly different, with OD600 of about 60 (see FIG. 3D, which is a diagram of the concentration of paclitaxel adiposomes measured by a multifunctional microplate reader, expressed as OD600).

1.3. Adiposomes encapsulating hydrophobic small molecule compound paclitaxel were prepared by altering the neutral lipid conditions, specifically the ratio of corn oil and tricaprylin (8:0TAG)

1.3.1) Neutral lipids were prepared according to the following 5 volume ratios, i.e., tricaprylin/corn oil = 1/0, 5/1, 4/1, 3/1, and 2/1.

The subsequent steps were the same as the preparation method in 1.1 above.

The obtained paclitaxel adiposomes were observed by laser confocal microscopy. Changing neutral lipid conditions, adiposomes encapsulating paclitaxel could also be prepared using corn oil and tricaprylin. The paclitaxel adiposomes presented a uniform spherical structure (see FIG. 4A, where a is the result of paclitaxel adiposomes stained red by the neutral lipid-specific dye LipidTox Red, and b is the DIC result of paclitaxel adiposomes, with a scale bar of 2 µm). The average particle sizes of paclitaxel adiposomes prepared with different neutral lipid volume ratios were not significantly different, about 150 nm (see FIG. 4B, which is a diagram of the average particle size of paclitaxel adiposomes measured by dynamic light scattering particle size analyzer). When the volume ratio of tricaprylin to corn oil was 3:1, the concentration of the prepared paclitaxel adiposomes was the highest, with an OD600 of about 80, while when the volume ratio of tricaprylin to corn oil was 2:1, the concentrations of the prepared paclitaxel adiposomes were the lowest, with an OD600 of about 20. Under other ratio conditions, the concentration of paclitaxel adiposomes was not significantly different, with OD600 of about 40 (see FIG. 4C, which is a diagram of the concentration of paclitaxel adiposomes measured by a multifunctional microplate reader, expressed as OD600).

1.4. Adiposomes encapsulating hydrophobic small molecule compound paclitaxel were prepared by altering the neutral lipid conditions, specifically the ratio of fish oil, tricaprylin, and retinyl ester

Neutral lipids were prepared according to the following 6 volume ratios, i.e., fish oil/tricaprylin/retinyl ester = 6/0/0, 4/1/1, 3/2/1, 3/1/2, 2/3/1, and 2/2/2.

The subsequent steps were the same as the preparation method in 1.1 above.

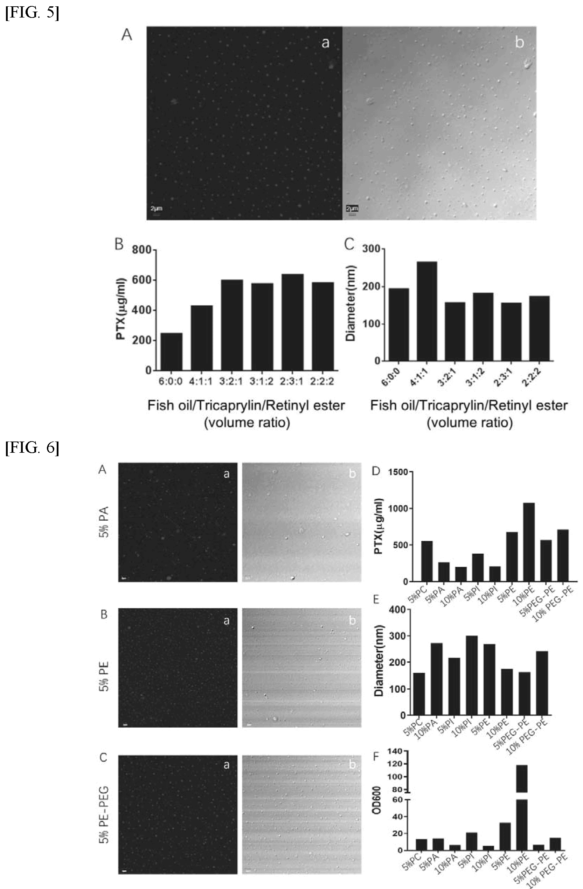

The obtained paclitaxel adiposomes were observed by laser confocal microscopy. The experimental results showed that changing neutral lipid conditions, adiposomes encapsulating paclitaxel could be prepared using fish oil, tricaprylin, and retinyl ester. The paclitaxel adiposomes presented a uniform spherical structure (see FIG. 5A, where a is the result of paclitaxel adiposomes stained red by the neutral lipid-specific dye LipidTox Red, and b is the DIC result of paclitaxel adiposomes, with a scale bar of 2 µm). The contents of PTX in paclitaxel adiposomes were measured by HPLC. Under conditions of fish oil, tricaprylin, and retinyl ester mixed at different ratios, the content of PTX in paclitaxel adiposomes was similar, reaching 600 µg/mL (see FIG. 5B, which is a diagram of the content of PTX in paclitaxel adiposomes measured by high performance liquid chromatography). The average particle sizes were not significantly different, about 200 nm (see FIG. 5C, which is a diagram of the average particle size of paclitaxel adiposomes measured by dynamic light scattering particle size analyzer).

1.5. Adiposomes encapsulating hydrophobic small molecule compound paclitaxel were prepared by altering the phospholipid conditions.

The neutral lipid conditions were kept unchanged, with fish oil and tricaprylin (1/1, volume ratio). The types and ratios of phospholipids were changed to prepare paclitaxel adiposomes. The following are the ratios and types of phospholipids used:
phosphatidyl choline (PC), phosphatidic acid (PA), phosphatidyl inositol (PI), phosphatidyl ethanolamine (PE), and PEGylation phosphatidyl ethanolamine (PEG-PE)

1.5.1) The types and ratios of phospholipids were changed, and phospholipid solutions were prepared with chloroform at a concentration of 32 mM. The types and ratios of phospholipids were altered according to the schemes in Table 2 below for the preparation of paclitaxel adiposomes.

**Table 2 Types and ratios of phospholipids for preparation of paclitaxel adiposomes**

| **Sample name** | **Phospholipid types and ratios (molar ratio)** |
|---|---|
| PC | 100 |
| 5% PA | PA:PC=5:95 |
| 10% PA | PA:PC=10:90 |
| 5% PI | PI:PC=5:95 |
| 10% PI | PI:PC=10:90 |
| 5% PE | PE:PC=5:95 |
| 10% PE | PE:PC=10:90 |
| 5% PEG-PE | PEG-PE:PC=5:95 |
| 10% PEG-PE | PEG-PE:PC=10:90 |

1.5.2) Fish oil and tricaprylin (1/1, volume ratio) were used to prepare neutral lipids containing PTX.

1.5.3) 80 µL of the above prepared phospholipids were taken and added to microcentrifuge tubes, respectively, and the solvent was dried by blowing with high-purity nitrogen gas.

1.5.4) 100 µL of PBS and 5 µL of the neutral lipids containing PTX prepared in step 1.5.2) were added to the microcentrifuge tubes.

The subsequent steps were the same as the preparation method in 1.1 above.

The obtained paclitaxel adiposomes were observed by laser confocal microscopy. The experimental results showed that adiposomes encapsulating paclitaxel could be prepared by changing phospholipid conditions, but different phospholipid ratios and types affected the morphology of paclitaxel adiposomes and the content of paclitaxel. Among them, paclitaxel adiposomes containing PA had relatively non-uniform morphology, while paclitaxel adiposomes with other phospholipids added presented a uniform spherical structure (see FIG. 6A to 6C, where a is the result of paclitaxel adiposomes stained red by the neutral lipid-specific dye LipidTox Red, and b is the DIC result of paclitaxel adiposomes, with a scale bar of 2 µm). The content of PTX in paclitaxel adiposomes prepared under different phospholipid conditions was measured by HPLC. The content of PTX in paclitaxel adiposomes under conditions of adding PA and PI was relatively low, about 300 µg/mL, while adding PE and PEG-PE significantly increased the content of PTX in paclitaxel adiposomes, which was more than 500 µg/mL (see FIG. 6D, which is a diagram of the content of PTX in paclitaxel adiposomes measured by high performance liquid chromatography). Adding PI, PE, and PEG-PE increased the average particle size of paclitaxel adiposomes, about 200 to 300 nm (see FIG. 6E, which is a diagram of the average particle size of paclitaxel adiposomes measured by dynamic light scattering particle size analyzer). When 10% of PE was added, the concentration of paclitaxel adiposomes was greatly increased, with an OD600 reaching 120, while the concentration of paclitaxel adiposomes prepared under other phospholipid conditions was about OD600 30 (see FIG. 6F, which is a diagram of the concentration of paclitaxel adiposomes measured by a multifunctional microplate reader, expressed as OD600).

### Example 2 Construction of adiposomes encapsulating hydrophobic small molecule compound honokiol (honokiol adiposomes)

According to the chemical structural formula of honokiol and the loading conditions of paclitaxel adiposomes in Example 1, the lipid conditions selected in this example were: phospholipid was 2-di-(9Z-octadecenoyl)-sn-glycero-3-phosphocholine (DOPC), neutral lipid was triolein (18:1TAG). Paclitaxel in Example 1 was replaced with honokiol, and the remaining operation methods were unchanged to prepare adiposomes encapsulating honokiol (denoted as honokiol adiposomes).

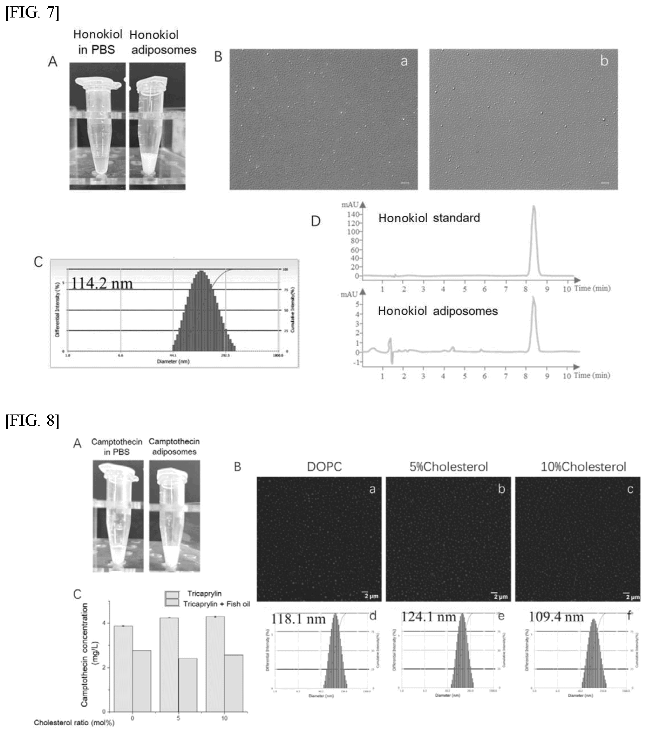

Due to its hydrophobicity, honokiol has very low solubility in PBS and presents a turbid state, while honokiol encapsulated in adiposomes presents a uniform milky white solution state (see FIG. 7A, which is a morphology picture of honokiol in PBS and adiposomes in PBS). Further, the prepared honokiol adiposomes were observed by laser microscopy. The honokiol adiposomes presented a uniform spherical structure (see FIG. 7B, where a is the result of honokiol adiposomes stained red by the neutral lipid-specific dye LipidTox Red, and b is the DIC result of honokiol adiposomes, with a scale bar of 5 µm). The average particle size of honokiol adiposomes was 114.2 nm (see -FIG. 7C, which is a diagram of the average particle size of honokiol adiposomes measured by dynamic light scattering particle size analyzer). The honokiol adiposomes were dissolved in methanol (chromatographic grade) for sample preparation, and the content of honokiol in the paclitaxel adiposomes was measured by HPLC. The detection conditions were: chromatographic column Agilent Zorbax SB-C18, loading volume 10 µL, mobile phase methanol:water = 7:3 (volume ratio), flow rate 1.0 mL/min, detection wavelength UV = 254 nm. The content of honokiol in honokiol adiposomes was about 17 µg/mL (see FIG. 7D, which is a diagram of the content of honokiol in honokiol adiposomes measured by high performance liquid chromatography).

### Example 3 Construction of adiposomes encapsulating hydrophobic small molecule compound camptothecin (camptothecin adiposomes)

According to the chemical structural formula of camptothecin and the loading conditions of paclitaxel adiposomes in Example 1, the lipid conditions selected in this example were: phospholipid was 2-di-(9Z-octadecenoyl)-sn-glycero-3-phosphocholine solution (DOPC) and cholesterol (Cho), neutral lipid was tricaprylin (8:0TAG) or a mixture of fish oil and tricaprylin (1:1 volume ratio). Paclitaxel in Example 1 was replaced with camptothecin, and the remaining operation methods were unchanged to prepare adiposomes containing camptothecin (denoted as camptothecin adiposomes).

Due to its hydrophobicity, camptothecin has very low solubility in PBS and presents a turbid state, while camptothecin encapsulated in adiposomes presents a uniform milky white solution state (see FIG. 8A, which is a morphology picture of camptothecin in PBS and adiposomes in PBS). Further, the prepared camptothecin adiposomes were observed by laser confocal microscopy. The camptothecin adiposomes presented a uniform spherical structure, and the average particle sizes of camptothecin adiposomes prepared under different phospholipid and neutral lipid conditions were similar, about 120 nm (see FIG. 8B, where a-c are the results of camptothecin adiposomes stained red by the neutral lipid-specific dye LipidTox Red, d-f are diagrams of the average particle size of camptothecin adiposomes measured by dynamic light scattering particle size analyzer, with a scale bar of 2 µm). The content of camptothecin in camptothecin adiposomes was measured by HPLC. Compared with the mixed neutral lipids of fish oil and tricaprylin, the content of camptothecin in camptothecin adiposomes prepared with only tricaprylin was relatively high, about 4 mg/L (see FIG. 8C, which is a diagram of the content of camptothecin in camptothecin adiposomes measured by high performance liquid chromatography).

### Example 4 Construction of adiposomes encapsulating hydrophobic small molecule compound auristatin derivative MMAE (Monomethyl auristatin E) (MMAE adiposomes)

According to the chemical structural formula of MMAE and the loading conditions of paclitaxel adiposomes in Example 1, the lipid conditions selected in this example were: phospholipid was 2-di-(9Z-octadecenoyl)-sn-glycero-3-phosphocholine solution (DOPC), neutral lipid was triolein (18:1TAG) or corn oil. Paclitaxel used in Example 1 was replaced with MMAE, and the remaining operation methods were unchanged to prepare adiposomes containing MMAE (denoted as MMAE adiposomes).

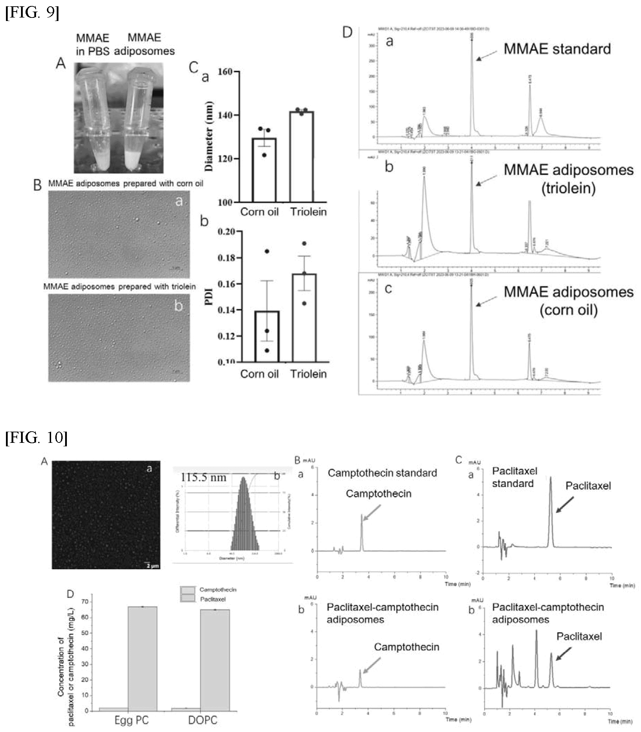

Due to its hydrophobicity, MMAE has very low solubility in PBS and presents a turbid state, while MMAE encapsulated in adiposomes presents a uniform milky white solution state (see FIG. 9A, which is a morphology picture of MMAE in PBS and adiposomes in PBS). Further, the prepared MMAE adiposomes were observed by optical microscopy. MMAE adiposomes prepared with corn oil or triolein both presented a uniform spherical structure (see FIG. 9B, where a is the DIC result of MMAE adiposomes prepared with corn oil, and b is the DIC result of MMAE adiposomes prepared with triolein, with a scale bar of 5 µm). In addition, the average particle sizes of MMAE adiposomes prepared with corn oil or triolein were similar, about 140 nm, and the polydispersity index (PDI) was less than 0.2 (see FIG. 9C, which is a diagram of the average particle size of MMAE adiposomes measured by dynamic light scattering particle size analyzer). 10 µL of MMAE adiposomes were taken and added to 190 µL of methanol (1% TCA), vortexed for 10 s, then centrifuged at 15000 rpm for 3 min. 150 µL of the supernatant was taken and added to a built-in sampling tube, and the content of MMAE in MMAE adiposomes was measured by HPLC. The detection conditions were: chromatographic column Agilent Zorbax SB-C18, loading volume 10 µL, mobile phase 0 min: 20% acetonitrile (1% TFA) - 80% water (1% TFA), 5 min: 80% acetonitrile (1% TFA) - 20% water (1% TFA), 7 min: 20% acetonitrile (1% TFA) - 80% water (1% TFA), flow rate 1.0 mL/min, detection wavelength UV = 210 nm. Consistent with the peak time of the MMAE standard, MMAE adiposomes prepared with corn oil or triolein showed MMAE signal at the same peak time. The content of MMAE in MMAE adiposomes prepared with corn oil was 100 µg/mL, and the content of MMAE in MMAE adiposomes prepared with triolein was 40 µg/mL (see FIG. 9D, which is a diagram of the content of MMAE in MMAE adiposomes measured by high performance liquid chromatography).

### Example 5 Construction of adiposomes simultaneously encapsulating hydrophobic small molecule compounds paclitaxel and camptothecin (paclitaxel-camptothecin adiposomes)

Lipid conditions: phospholipid was 2-di-(9Z-octadecenoyl)-sn-glycero-3-phosphocholine solution (DOPC) or egg phosphatidyl choline (Egg PC), neutral lipid was a mixed neutral lipid of fish oil and tricaprylin (1:1 volume ratio). Paclitaxel used in Example 1 was replaced with a mixture of paclitaxel and camptothecin (1:1 mass ratio), and the remaining operation methods were unchanged to prepare adiposomes simultaneously encapsulating hydrophobic small molecule compounds paclitaxel and camptothecin (paclitaxel-camptothecin adiposomes).

The prepared paclitaxel-camptothecin adiposomes were observed by laser confocal microscopy. The paclitaxel-camptothecin adiposomes presented a uniform spherical structure with an average particle size of 115.5 nm (see FIG. 10A, where a is the result of paclitaxel-camptothecin adiposomes stained red by the neutral lipid-specific dye LipidTox Red, and b is the average particle size of paclitaxel-camptothecin adiposomes measured by dynamic light scattering particle size analyzer, with a scale bar of 2 µm). Since the detection conditions for camptothecin and paclitaxel are different, the contents of camptothecin and paclitaxel in paclitaxel-camptothecin adiposomes were measured by HPLC according to the following methods, respectively. The HPLC detection conditions for camptothecin were: chromatographic column Agilent Zorbax SB-C18, mobile phase methanol:acetonitrile:water = 3:3:4 (volume ratio), column temperature 35°C, detection wavelength 366 nm, flow rate 0.8 mL/min. The detection conditions for paclitaxel were: chromatographic column Agilent Zorbax SB-C18, mobile phase methanol:acetonitrile:water = 4:3:3 (volume ratio), column temperature 25°C, detection wavelength 227 nm, flow rate 1 mL/min. When using the HPLC detection conditions for camptothecin, the peak time of camptothecin in paclitaxel-camptothecin adiposomes was consistent with the peak time of the camptothecin standard at 3.4 min, indicating that paclitaxel-camptothecin adiposomes successfully encapsulated camptothecin (see arrow in FIG. 10B, where a is the HPLC diagram of the camptothecin standard, and b is the HPLC diagram of camptothecin in paclitaxel-camptothecin adiposomes). Similarly, when using the HPLC detection conditions for paclitaxel, the peak time of paclitaxel in paclitaxel-camptothecin adiposomes was consistent with the peak time of the paclitaxel standard at 5.6 min, indicating that paclitaxel-camptothecin adiposomes successfully encapsulated paclitaxel (see arrow in FIG. 10C, where a is the HPLC diagram of the paclitaxel standard, and b is the HPLC diagram of paclitaxel in paclitaxel-camptothecin adiposomes). The content of camptothecin in paclitaxel-camptothecin adiposomes was about 4 mg/L, and the content of paclitaxel was about 65 mg/L (see D in FIG. 10, D is a diagram of the contents of paclitaxel and camptothecin in paclitaxel-camptothecin adiposomes prepared under Egg PC and DOPC conditions). The above results show that paclitaxel-camptothecin adiposomes successfully encapsulated both paclitaxel and camptothecin.

### Example 6 Adiposomes encapsulating hydrophobic small molecule compounds have biological activity and significantly kill cancer cells, and the killing effect is superior to that of free drugs

6.1. Paclitaxel adiposomes significantly kill cancer cells, and the killing effect is superior to that of free paclitaxel

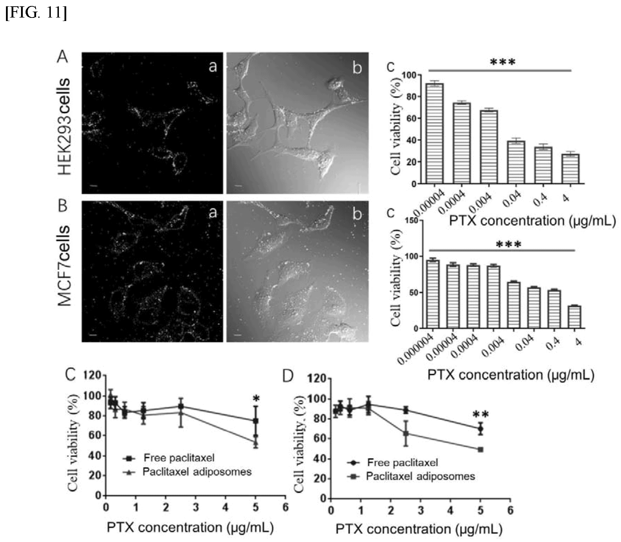

The constructed paclitaxel adiposomes were incubated with human embryonic kidney epithelial cells HEK293 for 60 minutes, then washed twice with saline, and observed by high-sensitivity laser scanning confocal microscopy to determine whether paclitaxel adiposomes could enter cells. The paclitaxel adiposomes were labeled with fluorescently labeled TAG, shown as green dots. The results showed that paclitaxel adiposomes could be taken up by cells and significantly killed HEK293 cells as paclitaxel content increased (see FIG. 11A, where a and b show paclitaxel adiposomes entering cells by high-sensitivity laser scanning confocal microscopy, and c is the cell viability after treatment with different concentrations of paclitaxel adiposomes). Similarly, the constructed paclitaxel adiposomes were incubated with human breast cancer cells MCF7 for 60 minutes. The paclitaxel adiposomes could be taken up by MCF7 cells and significantly killed MCF7 cells as paclitaxel content increased (see FIG. 11B, where a and b show paclitaxel adiposomes entering cells by high-sensitivity laser scanning confocal microscopy, and c is the cell viability after treatment with different concentrations of paclitaxel adiposomes). Furthermore, the effect of paclitaxel adiposomes and unencapsulated free drug paclitaxel on killing cancer cells was compared. The paclitaxel adiposomes prepared in 1.4 and 1.5 of Example 1 were used to treat human breast cancer cells MDA-MB-231, respectively. The results showed that compared with unencapsulated free paclitaxel drug, paclitaxel adiposomes had a better effect on killing cancer cells, especially when the concentration of paclitaxel was 5 µg/mL, the killing effect of paclitaxel adiposomes was significantly higher than that of free paclitaxel (see in FIG. 11C and 11D, where C is the viability diagram of paclitaxel adiposomes prepared in 1.4 of Example 1, and D is the viability diagram of paclitaxel adiposomes prepared in 1.5 of Example 1, * indicates p < 0.05, ** indicates p < 0.01).

Method for detecting cell viability: The cells to be treated, HEK293 or MCF7 or MDA-MB-231, were seeded in a 96-well plate at 1000 cells/well. After the cells were adherent overnight, the above cells were treated with different concentrations of drugs as shown in the figures and incubated for 72 hours. Subsequently, medium containing 10% CCK8 was added to replace the original medium. After incubation for 1 hour, absorbance at 450 nm was measured using a microplate reader, and cell viability was calculated by the following formula: $Cell viability = \left(Ae-Ab\right) / \left(Ac-Ab\right) * 100 %$
Wherein Ae represents the absorbance value of the well containing cells after drug treatment;
Ac represents the absorbance value of the well containing cells without drug treatment;
Ab represents the absorbance value of the blank well containing only medium and CCK8 reagent, used to correct background noise.

6.2. Honokiol adiposomes significantly kill cancer cells, and the killing effect is superior to that of free honokiol

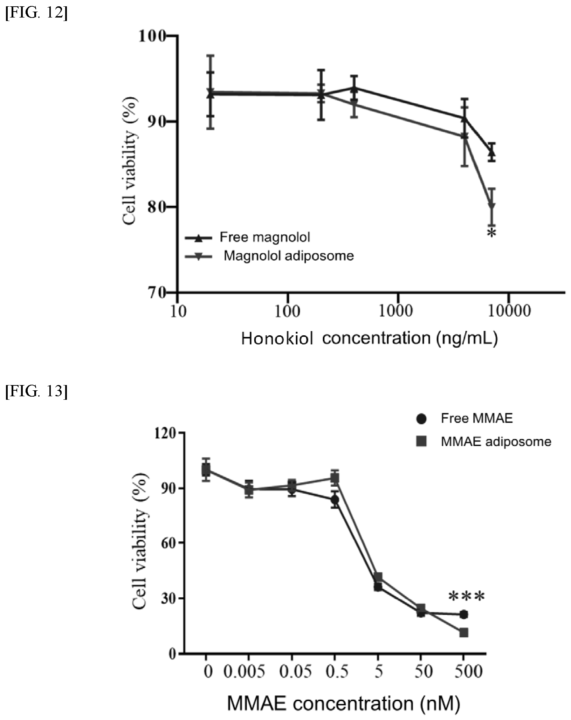

The experimental method was the same as in 6.1. The effect of honokiol adiposomes and unencapsulated free drug honokiol on killing cancer cells was compared. The honokiol adiposomes prepared in Example 2 were selected to treat human breast cancer cells MCF7. The results showed that compared with unencapsulated free honokiol drug, honokiol adiposomes had a better effect on killing cancer cells, especially when the concentration of honokiol was 10 µg/mL, the killing effect of honokiol adiposomes was significantly higher than that of free honokiol (see FIG. 12, which is a comparison diagram of viability of honokiol adiposomes and free honokiol, * indicates p < 0.05).

6.3. MMAE adiposomes significantly kill cancer cells, and the killing effect is superior to that of free MMAE

The experimental method was the same as in 6.1. The effect of MMAE adiposomes and unencapsulated free drug MMAE on killing cancer cells was compared. The MMAE adiposomes prepared in Example 4 were selected to treat mouse breast cancer cells 4T1. The results showed that compared with unencapsulated free MMAE drug, MMAE adiposomes had a better effect on killing cancer cells, especially when the concentration of MMAE was 500 nM, the killing effect of MMAE adiposomes was significantly higher than that of free MMAE (see FIG. 13, which is a comparison diagram of viability of MMAE adiposomes and free MMAE, *** indicates p < 0.001).

The above examples are only used to help understand the method of the present disclosure and its core idea. It should be pointed out that for those of ordinary skill in the art, without departing from the principles of the present disclosure, various improvements and modifications can be made to the present disclosure, and these improvements and modifications also fall within the scope of protection of the claims of the present disclosure.

## Claims

1. An adiposome nanoparticle encapsulating a hydrophobic small molecule drug, comprising a monolayer phospholipid membrane and a neutral lipid containing the hydrophobic small molecule drug, wherein the neutral lipid is encapsulated within the monolayer phospholipid membrane.

2. The adiposome nanoparticle encapsulating a hydrophobic small molecule drug according to claim 1, wherein the hydrophobic small molecule drug is selected from one or more of the group consisting of paclitaxel, honokiol, camptothecin, and auristatin derivative MMAE.

3. The adiposome nanoparticle encapsulating a hydrophobic small molecule drug according to claim 1, wherein the neutral lipid is selected from one or more of the group consisting of fish oil, corn oil, tricaprylin, and retinyl ester.

4. The adiposome nanoparticle encapsulating a hydrophobic small molecule drug according to claim 3, wherein the neutral lipid is selected from the group consisting of a mixture of fish oil and tricaprylin, a mixture of fish oil and retinyl ester, a mixture of tricaprylin and corn oil, and a mixture of fish oil, tricaprylin, and retinyl ester;
wherein the fish oil and the tricaprylin have a volume ratio of (1 to 5):(1 to 2);
the fish oil and the retinyl ester have a volume ratio of (1 to 5):(1 to 2);
the tricaprylin and the corn oil have a volume ratio of (1 to 5):(1 to 2); or
the fish oil, the tricaprylin, and the retinyl ester have a volume ratio of (2 to 4):(1 to 3):(1 to 2).

5. The adiposome nanoparticle encapsulating a hydrophobic small molecule drug according to claim 1, wherein the phospholipid membrane is selected from one or more of the group consisting of 2-di-(9Z-octadecenoyl)-sn-glycero-3-phosphocholine, phosphatidyl choline, phosphatidic acid, phosphatidyl inositol, phosphatidyl ethanolamine, and PEGylation phosphatidyl ethanolamine.

6. The adiposome nanoparticle encapsulating a hydrophobic small molecule drug according to claim 5, wherein the phospholipid membrane is selected from a mixture of phosphatidyl choline and any one of 2-di-(9Z-octadecenoyl)-sn-glycero-3-phosphocholine, phosphatidic acid, phosphatidyl inositol, phosphatidyl ethanolamine, or PEGylation phosphatidyl ethanolamine;
wherein a molar content of 2-di-(9Z-octadecenoyl)-sn-glycero-3-phosphocholine, phosphatidic acid, phosphatidyl inositol, phosphatidyl ethanolamine, or PEGylation phosphatidyl ethanolamine in the mixture is 5% to 10%.

7. A method for producing the adiposome nanoparticle encapsulating a hydrophobic small molecule drug according to any one of claims 1 to 6, comprising steps of:
S1) mixing a solution of a hydrophobic small molecule drug with a neutral lipid, and removing solvent to obtain a neutral lipid containing the hydrophobic small molecule drug;
S2) mixing the neutral lipid containing the hydrophobic small molecule drug with a phospholipid to obtain a lipid mixture, and performing separation to obtain the adiposome nanoparticle encapsulating the hydrophobic small molecule drug.

8. The method according to claim 7, wherein the step S2) is specifically:
A) vortexing the neutral lipid containing the hydrophobic small molecule drug and the phospholipid in a buffer to obtain a milky white lipid mixture;
B) repeatedly centrifuging and vortexing the lipid mixture, and performing separation to obtain the adiposome nanoparticle encapsulating the hydrophobic small molecule drug.

9. Use of the adiposome nanoparticle encapsulating a hydrophobic small molecule drug according to any one of claims 1 to 6 in manufacture of a medicament for preventing, alleviating, or treating a tumor, an infectious disease, and/or a metabolic disease.

10. A medicament for preventing, alleviating, or treating a tumor, an infectious disease, and/or a metabolic disease, comprising the adiposome nanoparticle encapsulating a hydrophobic small molecule drug according to any one of claims 1 to 6.
